# EUROPEAN PATENT APPLICATION

(11) **EP 4 082 489 A1**
(43) Date of publication of application: **02.11.2022**
(21) Application number: 21201540.8
(22) Date of filing: 01.10.2020
(51) Int. Cl.: A61F 2/46, A61B 34/30

(54) **IMPLANT POSITIONING TOOLS**

(30) Priority: 04.10.2019 US 201962910896 P
(62) Divisional of application: 20199702.0
(71) Applicant: Zimmer Biomet Spine, Inc., Westminster, Colorado 80021 (US)
(72) Inventor: LEQUETTE, Samuel, 33600 Pessac (FR); MILLARD, Thierry, 33610 Cestas (FR); PANNY, Anthony, 10800 Bucheres (FR); ICHELMANN, Bruno, 87100 Limoges (FR); JOUAN, Edouard, 10150 Lavau (FR)
(74) Representative: Mays, Julie

(57) **Abstract**

An instrument coupler can be receivable in an end effector coupler of a surgical arm, the instrument coupler can include a stem, a neck, and a guide. The stem can include a proximal portion couplable to the end effector coupler. The neck can be secured to the stem and can extend distally therefrom. The guide can be secured to the neck and can be configured to receive an implant positioning tool.

## Description

### CLAIM OF PRIORITY

This application claims the benefit of U.S. Provisional Patent Application Serial No. 62/910,896, filed on October 4, 2019, the benefit of priority of which is claimed hereby, and which is incorporated by reference herein in its entirety.

### BACKGROUND

The present invention relates generally to supported surgical instruments. Some surgical procedures include use of a variety of instruments. In some of these procedures, it is required to position components or implants using instruments during the procedure. Because it can be difficult or undesirable to manually position a component, mechanical and/or electromechanical arms can be used to hold or guide the position of the instrument and component. Some arms can be adjustable such that a position of the arm can be adjusted before or during the procedure.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1A illustrates an isometric view of a repositionable, lockable surgical arm system, in accordance with at least one example of this disclosure.
FIG. 1B illustrates an isometric view of a repositionable, lockable surgical arm system, in accordance with at least one example of this disclosure.
FIG. 2A illustrates an isometric view of an instrument coupler in accordance with at least one example of this disclosure.
FIG. 2B illustrates an isometric view of a trial positioning tool in accordance with at least one example of this disclosure.
FIG. 2C illustrates an isometric view of an implant positioning tool in accordance with at least one example of this disclosure.
FIG. 3A illustrates an isometric view of an instrument coupler and a trial positioning tool in a first condition, in accordance with at least one example of this disclosure.
FIG. 3B illustrates an isometric view of an instrument coupler and a trial positioning tool in a second condition, in accordance with at least one example of this disclosure.
FIG. 3C illustrates an isometric view of an instrument coupler and a trial positioning tool in a third condition, in accordance with at least one example of this disclosure.
FIG. 3D illustrates an isometric view of an instrument coupler, in accordance with at least one example of this disclosure.
FIG. 4A illustrates an isometric view of an instrument coupler, in accordance with at least one example of this disclosure.
FIG. 4B illustrates an isometric view of an instrument coupler and an implant positioning tool in a first condition, in accordance with at least one example of this disclosure.
FIG. 4C illustrates an isometric view of an instrument coupler and an implant positioning tool in a second condition, in accordance with at least one example of this disclosure.
FIG. 4D illustrates an isometric view of an instrument coupler and an implant positioning tool in a third condition, in accordance with at least one example of this disclosure.
FIG. 5A illustrates an isometric view of an instrument coupler, in accordance with at least one example of this disclosure.
FIG. 5B illustrates an isometric view of a trial positioning tool, in accordance with at least one example of this disclosure.
FIG. 5C illustrates an isometric view of an implant positioning tool, in accordance with at least one example of this disclosure.
FIG. 6 illustrates an isometric view of an instrument coupler, a trial positioning tool, and an implant positioning tool, in accordance with at least one example of this disclosure.
FIG. 7A illustrates an isometric view of an instrument adapter and a trial positioning tool in a first condition, in accordance with at least one example of this disclosure.
FIG. 7B illustrates an isometric view of an instrument coupler, an instrument adapter, and a trial positioning tool in a second condition, in accordance with at least one example of this disclosure.
FIG. 7C illustrates an isometric view of an instrument coupler, an instrument adapter, and a trial positioning tool in a third condition, in accordance with at least one example of this disclosure.
FIG. 7D illustrates an isometric view of an instrument coupler, in accordance with at least one example of this disclosure.
FIG. 8A illustrates an isometric view of an instrument coupler, in accordance with at least one example of this disclosure.
FIG. 8B illustrates an isometric view of an instrument coupler, an instrument adapter, and an implant positioning tool in a first condition, in accordance with at least one example of this disclosure.
FIG. 8C illustrates an isometric view of an instrument coupler, an instrument adapter, and an implant positioning tool in a second condition, in accordance with at least one example of this disclosure.
FIG. 8D illustrates an isometric view of an instrument coupler, an instrument adapter, and an implant positioning tool in a third condition, in accordance with at least one example of this disclosure.
FIG. 9A illustrates an isometric view of an instrument adapter and a portion of a trial positioning tool, in accordance with at least one example of this disclosure.
FIG. 9B illustrates an isometric view of an instrument adapter and a trial positioning tool with a portion of the instrument adapter shown in phantom, in accordance with at least one example of this disclosure.
FIG. 10A illustrates an isometric view of a portion of an instrument adapter and a portion of a trial positioning tool, in accordance with at least one example of this disclosure.
FIG. 10B illustrates an isometric view of a portion of an instrument adapter, in accordance with at least one example of this disclosure.
FIG. 11 illustrates an isometric view of an instrument coupler, an instrument adapter, and an implant positioning tool, in accordance with at least one example of this disclosure.
FIG. 12 illustrates an isometric view of an instrument coupler and an instrument adapter, in accordance with at least one example of this disclosure.
FIG. 13 illustrates an isometric view of a repositionable, lockable surgical arm, in accordance with at least one example of this disclosure.
FIG. 14 illustrates an isometric view of an instrument, in accordance with at least one example of this disclosure.
FIG. 15 illustrates a block diagram of a system, in accordance with at least one example of this disclosure.

### DETAILED DESCRIPTION

In some surgical procedures, it is required to position instruments, such as an implant tool, in a desired position, while an implant is secured to a part of a patient. In these procedures, adjustable mechanical and/or electromechanical arms can be used to hold the position of the instrument while other aspects of the procedure are performed. During a trialing and implantation process it may be required to change tools during the procedure, such as between the trialing tool and the implant tool. Further, it may be required to use multiple trial tools prior to selection of an implant size. In certain procedures, it can also be beneficial to maintain a particular trajectory for an instrument, but allow for movement along an axis along the trajectory.

This disclosure discusses methods, devices, and systems to save time during such a procedure by maintaining a positioning of a surgical arm to allow multiple tools to be quickly positioned in the same or similar location. For example, a surgical arm can include a coupler securable to an end effector coupler where the coupler is configured to receive multiple types of tools, such as a trial positioning tool and an implant positioning tool. The trial positioning tool can be used to find a desired location for a desired implant size. A position of the arm and coupler can then be locked allowing the implant positioning tool to be inserted into the coupler such that the implant can be quickly positioned to the previous location of the trial component. The coupler can be designed for relatively easy and quick coupling of the coupler to the trial and implant tools. Some couplers can include an adapter configured to use standard trial tools where the adapter is securable to the coupler.

The above discussion is intended to provide an overview of subject matter of the present patent application. It is not intended to provide an exclusive or exhaustive explanation of the invention. The description below is included to provide further information about the present patent application.

As used herein, the terms "proximal" and "distal" should be given their generally understood anatomical interpretation. The term "proximal" refers to a direction generally toward the torso of a patient or base or handle of a tool or instrument, and "distal" refers to the opposite direction of proximal, i.e., away from the torso of a patient or toward the working end of the tool.

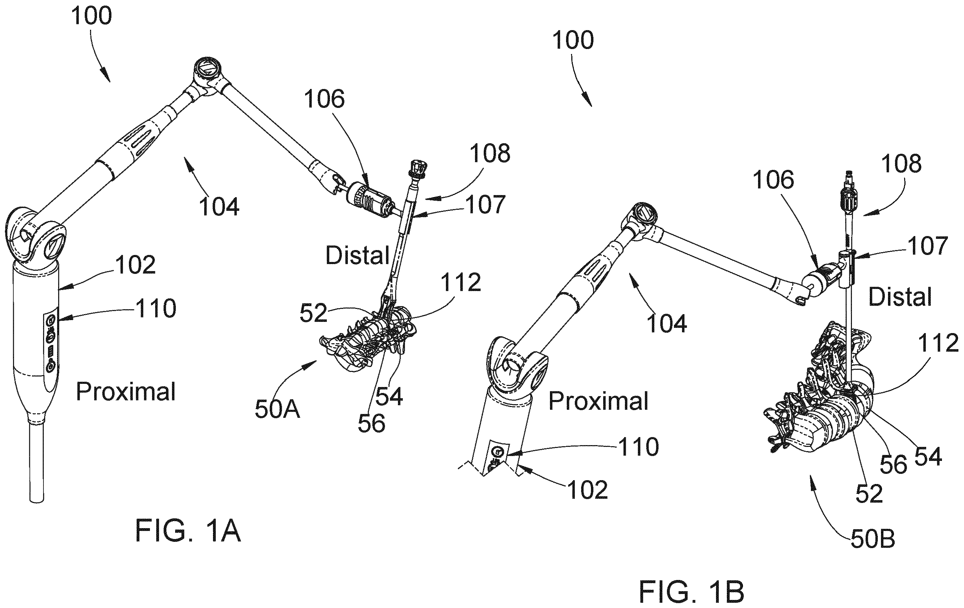

FIG. 1A illustrates an isometric view of a repositionable, lockable surgical arm system 100, in accordance with at least one example of this disclosure. FIG. 1B illustrates an isometric view of a repositionable, lockable surgical arm system 100, in accordance with at least one example of this disclosure. FIGS. 1A and 1B are discussed below concurrently.

The surgical arm 100 can include a control device 102, an arm 104, an end effector coupler 106, a guide 107, an instrument 108, and a user interface 110. The instrument 108 can be connected to an implant 112. Also shown in FIG. 1B are orientation indicators Proximal and Distal. Further shown in FIG. 1A is a cervical spine section 50A, including vertebra 52 and 54 and vertebral opening 56. Further shown in FIG. 1B is a lumber spine section 50B, including vertebra 52 and 54 and vertebral opening 56.

The control device 102, which can include power control circuit components for an electrically powered actuator, can be secured to a rail of a surgical table using, for example, a clamp. The user interface 110 of the control device can include buttons and/or switches for operating the surgical arm and can optionally include a screen that can display information about the status of the arm. In some examples, the arm 104 can include one or more locking joints and an actuator.

The end effector coupler 106 can be secured to the arm 104 and can be used to quickly and easily remove and secure tools and instruments to the arm 104, such as the guide 107. The guide 107 can be configured to receive standard or modified instruments for various procedures that can be performed using the lockable surgical arm system 104.

The instrument 108 can be an implant positioning tool configured to position an implant with respect to either the cervical spine section 50A or the lumbar spine section 50B. The user interface 110 can be configured to display information regarding the instrument 108 to a user and can be configured to receive inputs from a user in some examples. The user interface 110 is discussed in further detail below.

In operation of some examples, the user interface 110 can be operable by a user to initiate power locking and unlocking of the arm 104. When a lock/unlock button is not depressed, the arm 104 can be in a locked state where joints are locked such that proximal and distal links of the arm 104 cannot move relative to each other or to the table, the end effector coupler 106, or either the cervical spine section 50A or the lumbar spine section 50B. When the lock/unlock button is pressed, an actuator can unlock joints of the arm 104 such that the end effector coupler 106 and the instrument 108 can be positioned, as desired, and as guided by the arm 104. (In some examples, an actuator can be omitted and the arm joints can be individually lockable.) Re-locking the arm 104 will again lock the position of the arm 104. Such a process can be repeated, as desired. In some examples, the arm can include one or more actuators to move the arm 104 without force applied to the arm 104 by the user. For example, the joints of the arm 104 can include actuators in communication with the control unit 102.

Following or during positioning of the arm 104, the instrument 108 can be used, for example, to place an implant. In one example, the instrument 108 can be used position the implant 112 in the vertebral opening 56 between the vertebrae 52 and 54 during a fusion procedure. In such a procedure, the arm 104 can be positioned, as desired, with respect to the spine 50 (either cervical or lumbar) and the implant positioning tool 108 can be inserted into the guide 107. The guide 107 can guide translation of the implant positioning tool 108 to position the implant 112 in a desired position in the vertebral opening 56 between vertebrae 52 and 54. Further details regarding the guide 107, the instrument 108, and their use are discussed below.

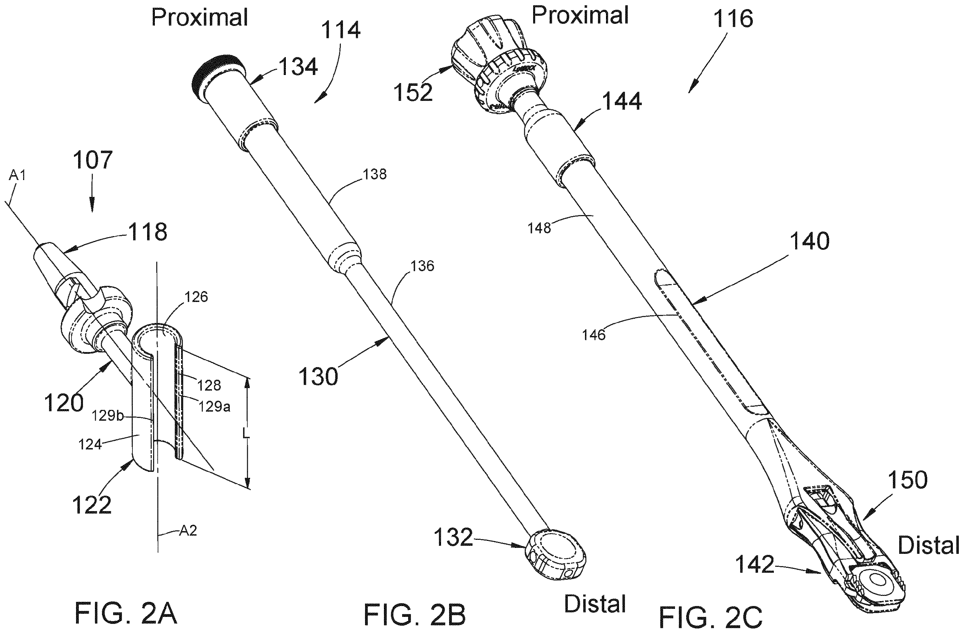

FIG. 2A illustrates an isometric view of an instrument coupler 107 in accordance with at least one example of this disclosure. FIG. 2B illustrates an isometric view of a trial positioning tool 114 in accordance with at least one example of this disclosure. FIG. 2C illustrates an isometric view of an implant positioning tool 116 in accordance with at least one example of this disclosure. FIGS. 2A-2C are discussed below concurrently. Also shown in FIG. 2A are axes A1 and A2. The instrument coupler 107 and tools (instruments) 114, 116 can form a system of instruments used by a surgeon to implant a prosthesis, such as an intervertebral implant. The instrument coupler 107 can be mounted on the arm 104. With the trial positioning tool 114 inserted into instrument coupler 107, the arm 104 can be locked into position when the trial implant 132 is in a desired position. After trialing is complete, the trial positioning tool 114 can be removed from the instrument coupler 107 without repositioning the arm 104. Then, the implant positioning tool 116 can be inserted into instrument coupler 107 and can be quickly and easily positioned in a similar position within the patient.

The coupler 107 shown in FIG. 2A can include a stem 118, a neck 120, and a guide 122. The stem 118 can include a proximal portion couplable to the end effector coupler 106 and a distal portion secured to the neck 120. The neck 120 can extend distally along axis A1 from the stem where the neck 120 can be coupled to the guide 122. The guide 122 can be secured to the neck 120 and can be configured to receive an implant positioning tool.

The guide 122 can include an elongate body 124 that can define a central axis of the body 124, or the axis A2. The body 124 can also define a bore 126 extending along the axis A2. The guide 122 can further define a slot 128 extending through the elongate body 124 of the guide 122 such that the slot 128 can intersect the bore 126. In some examples, the slot 128 can extend along an entirety of a length L of the guide. In some examples, the neck 120 and the stem 118 can be substantially coaxial with the axis A1. In some examples the bore 126 can be aligned with the axis A2, which can be substantially perpendicular to the neck 120 and/or the stem 118.

The trial positioning tool 114 shown in FIG. 2B can include a shaft 130, a trial implant 132, and a collar 134. The shaft 130 can include an insertion portion 136 and a retention portion 138. The trial implant 132 can be connected to a distal portion of the shaft 130, such as to the insertion portion 136. The insertion portion 136 can have a diameter sized for insertion through edges 129a and 129b of the slot 128 and into the central bore 126 of the guide 122. In some examples, the central bore 126 can be cylindrical (or substantially cylindrical) to receive the retention portion 138, which can have a complementary cylindrical shape. The retention portion 138 can be sized to translate within the bore 126 and sized to not pass through the slot 128. The collar 134 can be sized to contact a proximal portion of the guide 122 such that the collar 134 is larger than, and therefore cannot enter, the bore 126.

The implant positioning tool 116 shown in FIG. 2C can include a shaft 140, an implant 142, and a collar 144. The implant positioning tool 116 can also include a release 150 and an actuator 152. The shaft 140 can include an insertion portion 146 and a retention portion 148. The implant 142 can be connected to a distal portion of the shaft 140, such as to the insertion portion 146, through the release 150. The insertion portion 146 can have a diameter sized for insertion through edges 129a and 129b of the slot 128 and into the central bore 126 of the guide 122. The retention portion 148 can be sized to translate within the bore 126 and sized to not pass through the slot 128. The collar 144 can be sized to contact a proximal portion of the guide 122 such that the collar 144 is larger than, and therefore cannot enter, the bore 126. The release 150 can be located at a distal portion of the shaft 140 and can releasably secure the implant 142 to the shaft 140. The release 150 can be operated to release the implant by the actuator 152. The actuator 152 can be located on a proximal portion of the shaft 140. Further details and operation of the coupler 107, the trial positioning tool 114, and the implant positioning tool 116 are discussed below.

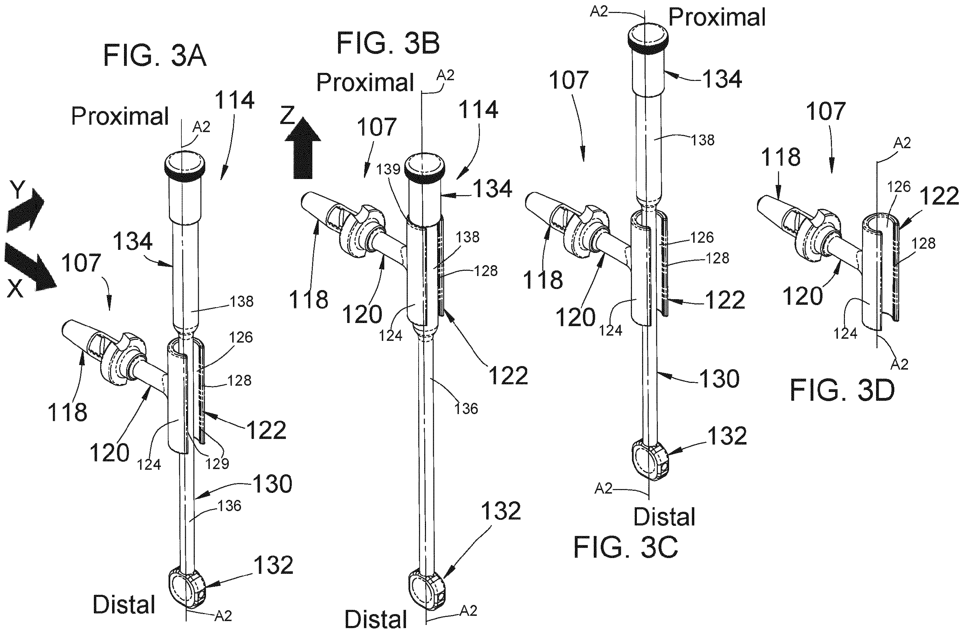

FIG. 3A illustrates an isometric view of the instrument coupler 107 and the trial positioning tool 114 in a first condition, in accordance with at least one example of this disclosure. FIG. 3B illustrates an isometric view of the instrument coupler 107 and the trial positioning tool 114 in a second condition, in accordance with at least one example of this disclosure. FIG. 3C illustrates an isometric view of the instrument coupler 107 and the trial positioning tool 114 in a third condition, in accordance with at least one example of this disclosure. FIG. 3D illustrates an isometric view of the instrument coupler 107, in accordance with at least one example of this disclosure. FIGS. 3A-3C show orientation indicators Proximal and Distal. FIGS. 3A-3D show axis A2. FIG. 3A shows directional arrows X and Y and FIG. 3B shows directional arrow Z. FIGS. 3A-3D are discussed below concurrently.

FIGS. 3A-3D generally show an example of a method of using the coupler 107 to position the trial positioning tool 114 with respect to a spine, such as either the cervical spine section 50A or the lumbar spine section 50B of FIG. 1. The steps or operations of the method are illustrated in a particular order for convenience and clarity; many of the discussed operations can be performed in a different sequence or in parallel without materially impacting other operations. The method as discussed includes operations performed by multiple different actors, devices, and/or systems. It is understood that subsets of the operations discussed in the method can be attributable to a single actor, device, or system could be considered a separate standalone process or method.

In FIG. 3A, the trial positioning tool 114 is shown as positioned within the coupler 107, the insertion portion 136 having been received through the edges 129 of the slot 128 and into the bore 126 of the guide 122 such that the shaft 130 is aligned with the axis A2. At this point, the coupler 107, together with a surgical arm (such as the arm 104 of FIG. 1) can be moved to position the implant 132 in the X direction. The coupler 107 can also be moved in the Y direction during this step. Either before or after the implant is positioned in a desired X and Y position, the trial positioning tool 114 can be translated along the A2 axis until the collar 134 contacts a proximal portion 139 of the guide 122 to limit the translation. In this position, the retention portion 138 of the shaft 130 can be positioned within the bore 126 to limit movement of the shaft 130 in the X and Y directions with respect to the guide 122. That is, when the retention portion 138 of the shaft 130 is positioned within the bore 126, the trial positioning tool 114 can be moved only in the Z direction (or along the axis A2) with respect to the guide 122.

As shown in FIG. 3B, the coupler 107 (together with the arm) can be moved in the Z direction with respect to the spine to position the trial implant 132 as desired. Multiple trial positioning tools having implants of different sizes can be inserted into the guide 122 until an implant size is selected. When the collar 134 is in contact with the guide 122 and when the trial implant 132 is positioned as desired with respect to the spine in the X, Y, and Z directions, a position of the coupler (and the arm) can be locked. Then, as shown in FIG. 3C, with the position of the coupler (and the arm) locked, additional trial tools can be tested. Further, with the coupler 107 locked, imaging of the surgical area, such as x-rays, can be performed to determine whether the position of the trial implant 132 with respect to the spine is desirable.

With the position of the coupler (and the arm) 107 remaining locked, the trial positioning tool 114 can be translated proximally in the Z direction along the axis A2 and then trial positioning tool 114 can be removed from the guide 122 through the slot 128, such that the guide 122 remains locked in position, as shown in FIG. 3D.

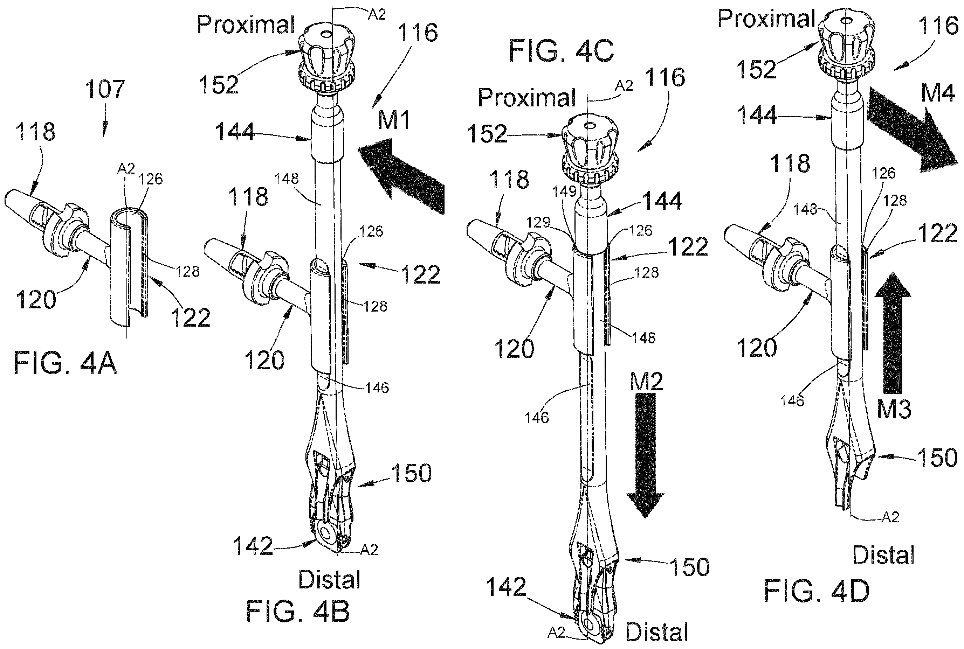

FIG. 4A illustrates an isometric view of the instrument coupler 107, in accordance with at least one example of this disclosure. FIG. 4B illustrates an isometric view of the instrument coupler 107 and the implant positioning tool 116 in a first condition, in accordance with at least one example of this disclosure. FIG. 4C illustrates an isometric view of the instrument coupler 107 and the implant positioning tool 116 in a second condition, in accordance with at least one example of this disclosure. FIG. 4D illustrates an isometric view of the instrument coupler 107 and the implant positioning tool 116 in a third condition, in accordance with at least one example of this disclosure. FIGS. 4A-4D are discussed below concurrently.

FIGS. 4B-4D show orientation indicators Proximal and Distal. FIGS. 4A-4D show axis A2. FIG. 4B shows movement arrow M1, FIG. 4C shows movement arrow M2, and FIG. 4D shows movement arrows M3 and M4. FIGS. 4A-4D generally show an example of a method of using the coupler 107 to position the implant positioning tool 116 with respect to a spine, such as either of the spines 50A and 50B of FIGS. 1 and 1B. The steps of the method shown in the FIGS. 4A-4D can be continued from the steps discussed with respect to FIGS. 3A-3D or steps of the method shown in the FIGS. 4A-4D can be performed independently.

As shown in FIG. 4A, a position of the coupler 107 can be fixed or in a locked position. In some examples, the position of the coupler 107 can be selected and fixed using the steps discussed with respect to FIGS. 3A-3D to determine a position of the guide 122 that will position the implant 142 in a desired location with respect to the spine.

With the position of the guide 122 locked, the implant positioning tool 116 can be inserted into the guide 122 by moving the implant positioning tool 116 in the direction M1 and by passing the insertion portion 146 between the edges 129 of the slot 128 and into the bore 126 of the guide 122 such that the shaft 140 is aligned with the axis A2.

Then, as shown in FIG. 4C, the implant positioning tool 116 can be moved in the direction M2 (the Z direction) such that the implant positioning tool 116 translates along the axis A2 until the collar 144 contacts the proximal portion 139 of the guide to limit translation of the implant positioning tool 116 within the guide and to position the implant 142 in the desired location with respect to the spine. During translation, the retention portion 148 of the shaft 140 is positioned within the bore 126, limiting movement of the implant positioning tool 116 in the X and Y directions (and limiting movement of the implant positioning tool 116 to movement along the axis A2) with respect to the guide 122. In some examples, with the implant 142 in the desired position, imaging of the surgical area, such as x-rays, can be performed to determine whether the position of the implant 142 with respect to the spine is desirable. If desired, the surgical arm can be moved to make small adjustments to the position of the implant 142, as required. The implant 142 can then be partially or completely fixed to adjacent vertebrae with the coupler 107 in the locked position.

When it is confirmed that the implant 142 is in the desired position, the actuator 152 can be operated to actuate the release 150 to release the implant 142 from the shaft 140. Securing of the implant 142 can be completed after release, if not completed prior to release. After release of the implant 142 from the implant positioning tool 116, the implant positioning tool 116 can be translated along the axis A2 in the direction M3, as shown in FIG. 4D. When the insertion portion 146 aligns with the slot 128, the implant positioning tool 116 can be moved in the direction M4 through the slot 128 to remove the implant positioning tool 116 from the guide 122. Thereafter, the procedure can be completed.

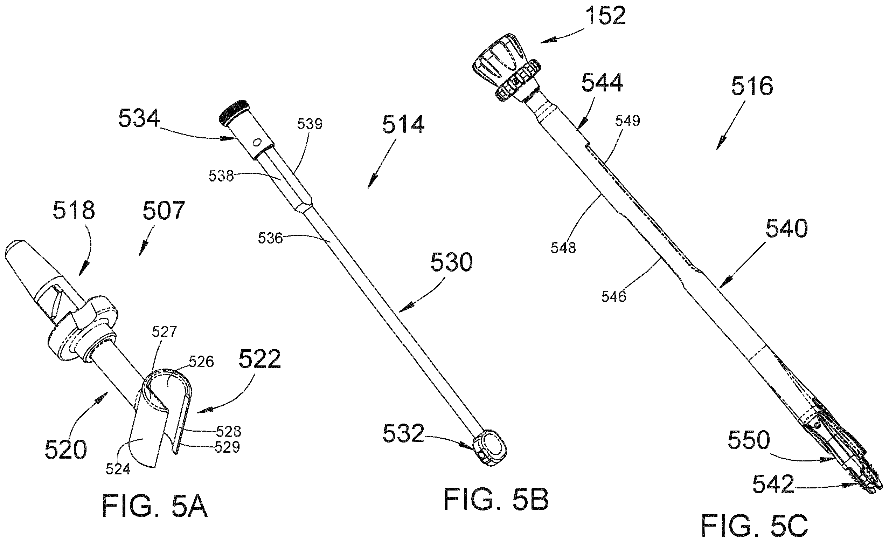

FIG. 5A illustrates an isometric view of an instrument coupler 507, in accordance with at least one example of this disclosure. FIG. 5B illustrates an isometric view of a trial positioning tool 514, in accordance with at least one example of this disclosure. FIG. 5C illustrates an isometric view of an implant positioning tool 516, in accordance with at least one example of this disclosure. FIGS. 5A-5C are discussed below concurrently.

The coupler 507, the trial positioning tool 514, and the implant positioning tool 516 can be similar to the coupler, 107, the trial positioning tool 114, and the implant positioning tool 116, respectively, discussed above, except that a guide 522 of the coupler 507 can include a bore 526 having a flat 527. The flat 527 can be complimentary to a flat 539 of the trial positioning tool 514 and can be complimentary to a flat 549 of the implant positioning tool 516.

The flat 527 of the guide 522 can orient the trial positioning tool 514 with respect to the guide 522, a stem 518, and an end effector coupler when a retention portion 538 of a shaft 530 is position in the bore 526 and when the flat 539 engages the flat 527 of the bore 526. Similarly, the flat 527 of the guide 522 can orient the implant positioning tool 516 with respect to the guide 522, the stem 518, and an end effector coupler when the retention portion 548 of a shaft 540 of the implant positioning tool 516 is position in the bore 526 and when the flat 549 engages the flat 527 of the bore 526. The flat 527 can also help limit rotation of the trial positioning tool 514 and the implant positioning tool 516 when the flat 539 engages the flat 527 and when the flat 549 engages the flat 527, respectively.

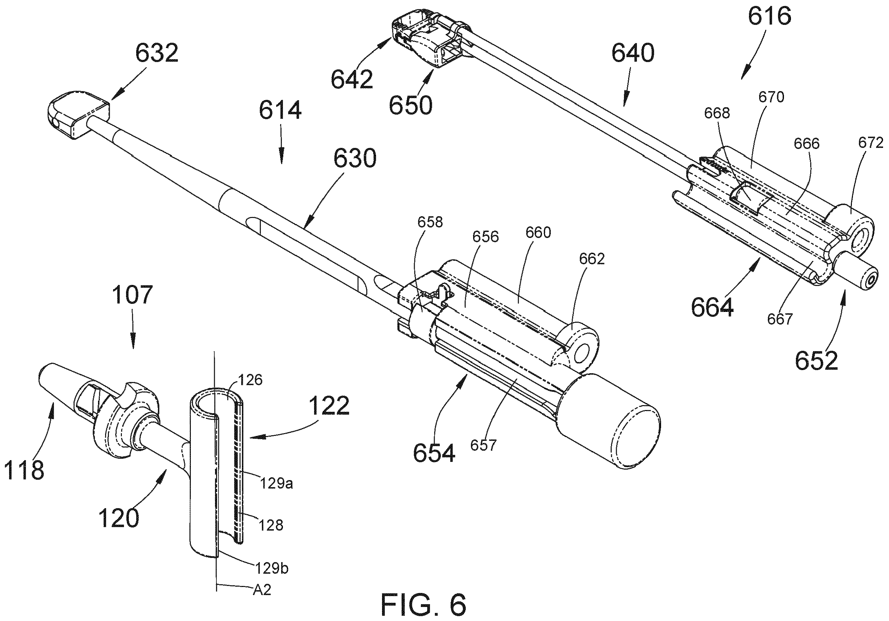

FIG. 6 illustrates an isometric view of an instrument coupler 107, a trial positioning tool 614, and an implant positioning tool 616, in accordance with at least one example of this disclosure. Also shown in FIG. 6 is axis A2.

The instrument coupler 107 can be consistent with the instrument coupler 107 discussed above. The trial positioning tool 614 can be similar to the trial positioning tool 114 except that the trial positioning tool 614 can include an adapter 654. The adapter 654 can include a body 656 (including a body bore) 657, a locking mechanism (or lock) 658, a pin 660, and a collar 662. Also, the implant positioning tool 616 can be similar to the implant positioning tool 116 except that the implant positioning tool 616 can include an adapter 664. The adapter 664 can include a body 666 (including a body bore 667), a locking mechanism (or lock) 668, a pin 670, and a collar 672.

With regard to the adapter 654, the body 656 can be a rigid or semi-rigid member configured to support the components of the adapter 654. The locking mechanism 658 can be connected to the body 656 and can be configured to secure the shaft 630 of the trial positioning tool 614 within the body bore 657 of the adapter 654. The pin 660 can be connected to the body 656 and sized and shaped to be insertable into the bore 126 of the guide 122. The collar 662 can be connected to a proximal portion of the pin 660 and the body 654. The collar 662 and can have a diameter larger than the pin 660 to limit translation of the pin 660 into the bore 126 of the guide 122.

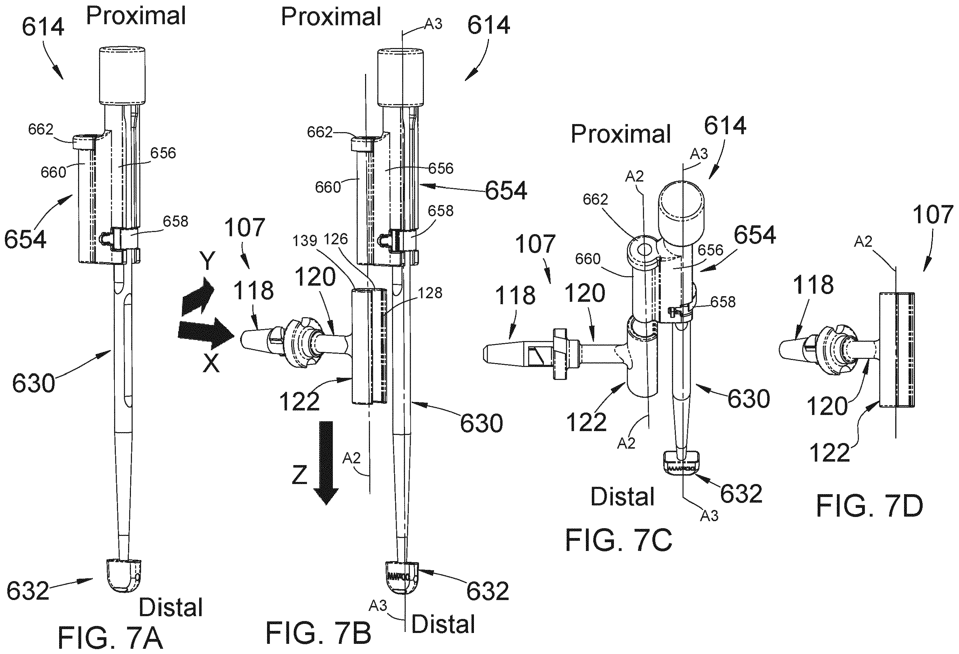

With regard to the adapter 664, the body 666 can be a rigid or semi-rigid member configured to support the components of the adapter 664. The locking mechanism 668 can be connected to the body 666 and can be configured to secure the shaft 640 of the implant positioning tool 616 within the body bore 667 of the adapter 654. The pin 670 can be connected to the body 666 and sized and shaped to be insertable into the bore 126 of the guide 122. The collar 672 can be connected to a proximal portion of the pin 670 and can have a diameter larger than the pin 670 to limit translation of the pin 670 into the bore 126 of the guide 122. The adapters 654 and 664 are discussed further with respect to FIGS. 7A-8D below.

FIG. 7A illustrates an isometric view of the instrument adapter 654 and the trial positioning tool 614 in a first condition, in accordance with at least one example of this disclosure. FIG. 7B illustrates an isometric view of the instrument coupler 107, the instrument adapter 654, and the trial positioning tool 614 in a second condition, in accordance with at least one example of this disclosure. FIG. 7C illustrates an isometric view of the instrument coupler 107, the instrument adapter 654, and the trial positioning tool 614 in a third condition, in accordance with at least one example of this disclosure. FIG. 7D illustrates an isometric view of the instrument coupler 107, in accordance with at least one example of this disclosure. FIGS. 7A-7D are discussed below concurrently. FIGS. 7A-7C show orientation indicators Proximal and Distal. FIGS. 7B-7D show axis A2. Fig. 7B shows directional arrows X, Y, and Z.

FIG. 7A shows the trial positioning tool 614 oriented for insertion into the adapter 654. Then, FIG. 7B shows that the coupler 107 and the trial positioning tool 614 can be positioned, for example, with respect to a spine and the pin 660 can be inserted into the bore 126 of the guide 122 to secure the adapter 654 (and therefore the trial positioning tool 614) to the guide 122 (and therefore to a surgical arm, such as the arm 104). Then, the coupler 107, together with a surgical arm (such as the arm 104 of FIG. 1) can be moved to position the implant 632 in the X and Y directions. Either before or after the implant is positioned in a desired X and Y position, the trial positioning tool 614 can be translated along the A2 axis until the collar 662 contacts the proximal portion 139 of the guide 122 to limit translation of the adapter 654 with respect to the guide 107. In this position, the pin 660 can be positioned within the bore 126 to limit movement of the adapter 654 in the X and Y directions with respect to the guide 122. That is, when the pin 660 is positioned within the bore 126, the trial positioning tool 614 can be moved only in the Z direction (or along the axis A2) with respect to the guide 122.

As shown in FIG. 7B, the coupler 107 (together with the arm) can also be moved in the Z direction, along an axis A3 of the slot 657, with respect to the spine to position the trial implant 132 as desired. In some examples, the axis A3 can substantially parallel with the axis A2 of the guide 122 and in some examples, the axis A3 can be substantially perpendicular to the stem 118. Multiple trial positioning tools having implants of different sizes can be inserted into the guide 122 until an implant size is selected. When the trial implant 632 is positioned as desired with respect to the spine in the X, Y, and Z directions, a position of the coupler 107 (and the arm) can be locked. Further, with the coupler 107 locked, imaging of the surgical area, such as x-rays, can be performed to determine whether the position of the trial implant 132 with respect to the spine is desirable.

With the position of the coupler (and the arm) 107 remaining locked, the trial positioning tool 614 can be translated in the Z direction along the axis A2 and the pin 660 can be removed from the guide 122 to disengage the trial positioning tool 614 from the guide 122 such that the guide 122 remains locked in position, as shown in FIG. 7D.

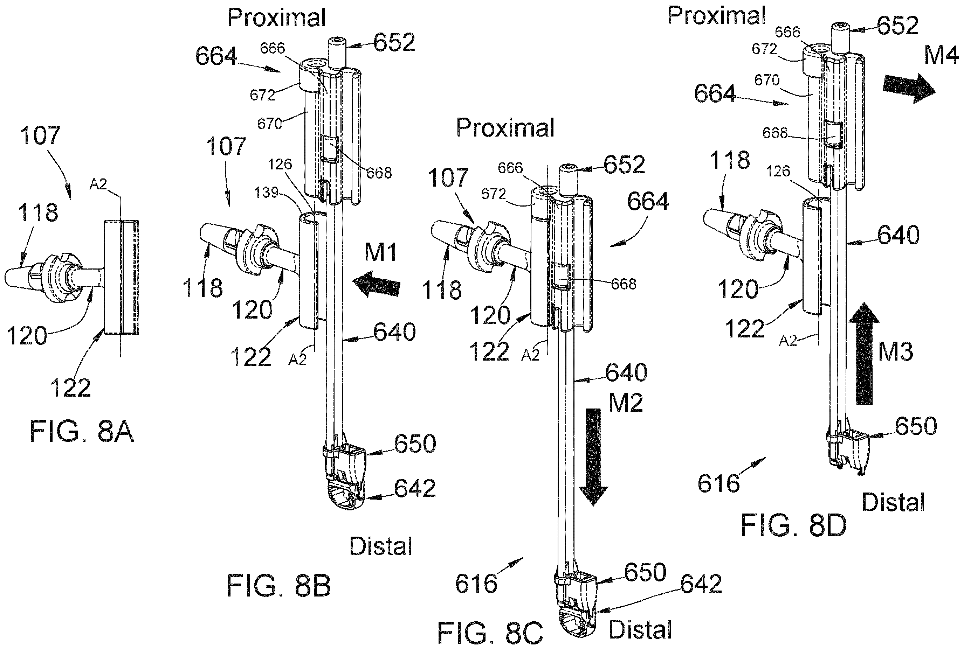

FIG. 8A illustrates an isometric view of the instrument coupler 107, in accordance with at least one example of this disclosure. FIG. 8B illustrates an isometric view of the instrument coupler 107, the instrument adapter 664, and the implant positioning tool 616 in a first condition, in accordance with at least one example of this disclosure. FIG. 8C illustrates an isometric view of the instrument coupler 107, the instrument adapter 664, and the implant positioning tool 616 in a second condition, in accordance with at least one example of this disclosure. FIG. 8D illustrates an isometric view of the instrument coupler 107, the instrument adapter 664, and the implant positioning tool 616 in a third condition, in accordance with at least one example of this disclosure. FIGS. 8A-8D are discussed below concurrently.

FIGS. 8B-8D show orientation indicators Proximal and Distal. FIGS. 8A-9D show axis A2. Fig. 8B shows movement arrow M1, FIG. 8C shows movement arrow M2, and FIG. 8D shows movement arrows M3 and M4. FIGS. 8A-8D generally show an example of a method of using the coupler 107 to position the implant positioning tool 616 with respect to a spine, such as either of the spines 50A and 50B of FIGS. 1A and 1B. The steps of the method shown in the FIGS. 8A-8D can be continued from the steps discussed with respect to FIGS. 7A-7D or steps of the method shown in the FIGS. 8A-8D can be performed independently.

As shown in FIG. 8A, a position of the coupler 107 can be fixed or in a locked position. In some examples, the position can be selected using the steps discussed with respect to FIGS. 7A-7D to determine a position of the guide that will position the implant 642 in a desired location with respect to the spine. As shown in FIG. 8B, with the position of the guide 122 locked, the implant positioning tool 616 can be secured to the guide 122 by moving the implant positioning tool 616 in the direction M1 and by moving the adapter 666 to a position where the pin 670 is aligned with the axis A2.

Then, as shown in FIG. 8C, the implant positioning tool 616 can be moved in the direction M2 (the Z direction) such that the implant positioning tool 616 translates along the axis A2 until the collar 672 contacts the proximal portion 129 of the guide 122 to limit translation of the pin 670 within the guide 122 and to position the implant 642 in the desired location with respect to the spine. During translation, the pin 670 can be positioned within the bore 126, limiting movement of the implant positioning tool 616 in the X and Y directions (limiting movement of the implant positioning tool 616 to movement along the axis A2) with respect to the guide 122. In some examples, with the implant 642 in the desired position, imaging of the surgical area, such as x-rays, can be performed to determine whether the position of the implant 642 with respect to the spine is desirable. The surgical arm can be used to make small adjustments to the position of the implant 642, as desired. The implant 642 can be partially or completely fixed to adjacent vertebra with the coupler 107 in the locked position.

When it is confirmed that the implant 642 is in the desired position, the actuator 652 can be operated to actuate the release 650 to release the implant 642 from the shaft 640. Securing of the implant 642 can be completed after release, if not completed prior to release. After release of the implant 642 from the implant positioning tool 616, the implant positioning tool 616 can be translated along the axis A2 in the direction M3, as shown in FIG. 8D. When the pin 670 is removed from the bore 126 of the guide 122, the implant positioning tool 616 can be moved in the direction M4. Thereafter, the procedure can be completed.

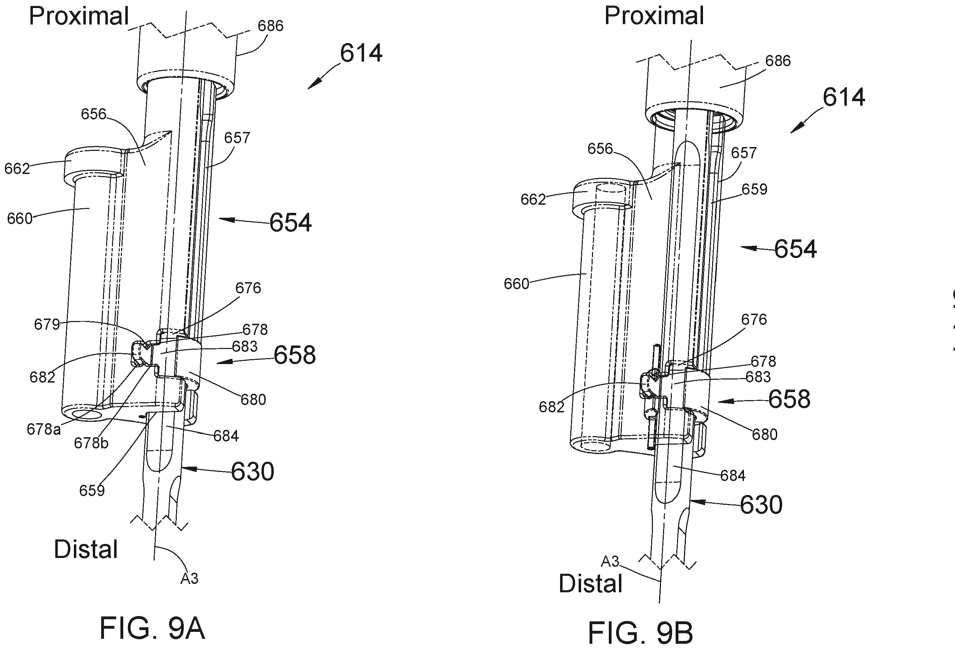

FIG. 9A illustrates an isometric view of an instrument adapter 654 and a portion of a trial positioning tool 614, in accordance with at least one example of this disclosure. FIG. 9B illustrates an isometric view of the instrument adapter 654 and the trial positioning tool 614 with a portion of the instrument adapter shown in phantom, in accordance with at least one example of this disclosure. FIGS. 9A and 9B are discussed below concurrently. FIGS. 9A and 9B show orientation indicators Proximal and Distal and axis A3.

The instrument adapter 654 and the trial positioning tool 614 of FIGS. 9A and 9B can be consistent with FIGS. 6A-8D discussed above; further details of the instrument adapter 654 and the trial positioning tool 614 are discussed with respect to FIGS. 9A and 9B. For example, FIGS. 9A and 9B show that the body 656 can include the slot 657, which can be a slot extending proximally-to-distally along the body 656 and can be sized to receive an insertion portion 684 therethrough. The slot 657 can extend along an entirety of a length of the body 656 and can intersect a central bore 659 which can be substantially cylindrical. The central bore 659 can define the axis A3. FIG. 9A also shows that a knob 686 can be connected to the shaft 630 of the trial positioning tool 614 and can have a diameter relatively larger than the shaft 630 such that the knob 686 can engage a proximal portion of the adapter 654 to limit proximal-to-distal translation of the shaft 630 with respect to the adapter 654.

FIGS. 9A and 9B also show that the body 656 can include a lock ring slot 676. The lock ring slot 676 can be an undercut or slot extending from the slot 657 into the body 656. The lock ring slot 676 can be configured to support at least a portion of a lock ring 680 of the lock 658. The body 656 can further include a notch 678 adjacent to the lock ring slot 676 and configured to support at least a portion of a lock ring 680. The lock ring slot 676 can be adjacent and connected to the notch 678, which can include a first portion 678a and a second portion 678b separated by a tooth 679. The first portion 678a and the second portion 678b can each be sized smaller than the lock ring slot 676 and sized to receive a tab 682 of the lock ring 680.

FIGS. 9A and 9B also show that the lock 658 can further include the lock ring 680, which can be substantially cylindrical and can include the tab 682 that extends radially from the lock ring 680 and can include a gap 683 in the lock ring, which can be a circumferential gap of the lock ring 680. The lock ring 680 can be positioned in the lock ring slot 676 and can be secured therein. The lock ring 680 can be rotatable (at least partially) within the lock ring slot 676 about the central bore 659.

In operation, the lock ring 680 can be rotated within the lock ring slot 676 so that the gap 683 of the lock ring 680 aligns with the slot 657 such that the lock ring 680 is in an unlocked position. The insertion portion 684 of the shaft 630 can be inserted through the gap 683 of the lock ring 680 and the slot 657 into the central bore 659. Once the insertion portion 684 of the shaft 630 is secured within the central bore 659, the lock ring 680 can be rotated within the lock ring slot 676 such that the lock ring 680 spans the slot 657 (the gap 683 does not align with the slot 657). In this position, the tab 682 can be moved past the tooth 679 into the first portion 678a to lock a position of the tab 682 and therefore the lock ring 680. The tooth 679 can be a smaller size than the first portion 678a and the second portion 678b to limit movement of the tab 682 from the first portion 678a of the notch 678 when the lock ring 680 is in the locked position in the lock ring slot 676, which can help to limit rotation of the lock ring 680 within the lock ring slot 676 when the lock ring 680 is in the locked position in the lock ring slot 676. When the lock ring 680 is in the locked position, the lock ring 680 can limit movement of the shaft 630 from the slot 657, helping to limit movement of the shaft 630 to translation along the axis A3 of the central bore 659.

When it is desired to remove or uncouple the shaft 630 from the instrument adapter 654, the tab 682 of the lock ring 680 can be forced over the tooth 679 to the second portion 678b of the notch 678 so that the lock ring 682 can be rotated to the unlocked position such that the lock ring gap 683 aligns with the slot 657 of the body 656 of the instrument adapter 654. The shaft 630 can then be removed from the body 656 through the slot 657.

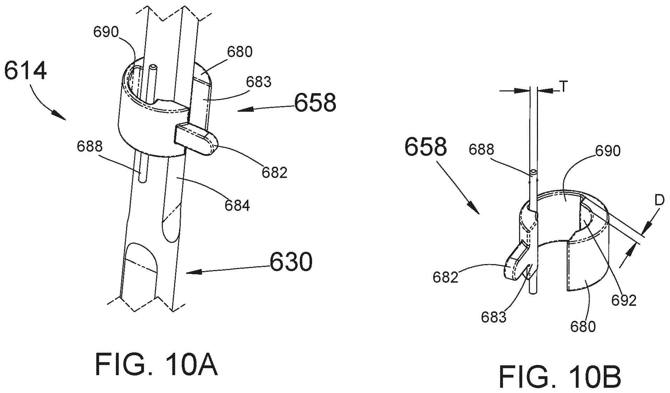

FIG. 10A illustrates an isometric view of a portion of the lock 658 and a portion of the trial positioning tool 614, in accordance with at least one example of this disclosure. FIG. 10B illustrates an isometric view of the lock 658, in accordance with at least one example of this disclosure. FIGS. 10A and 10B are discussed below concurrently.

The lock 658 and the trial positioning tool 614 can be consistent with FIGS. 6, 7A-7D, and 9A-9B discussed above; further details of these components are discussed below. For example, FIG. 10A shows that the shaft 630 of the trial positioning tool 614 can include a pin 688, which can be a relatively small diameter pin secured to an outer surface of the shaft 630 and having a relatively short axial length. The pin 688 can extend radially outward from the shaft 630 and can be substantially cylindrical, but can be other shapes such as a rectangular prism.

FIGS. 10A and 10B also show that the lock ring 680 can include a channel 690, which can be a portion of the lock 680 with a reduced radial thickness to create a circumferentially extending channel in the lock ring 680. The channel 690 can extend around only a portion of the lock 680, in some examples. The channel 690 can have a depth D substantially equivalent to a thickness T of the pin 688 such that the pin 688 can be positioned within the channel 690 when the shaft 630 is positioned within a bore 692 of the lock ring 680. In operation, when the shaft 630 is inserted into the bore 692 of the lock ring 680, walls of the channel 690 can be engageable with the pin 688 to limit rotation of the lock ring 680 to between the locked position and the unlocked position.

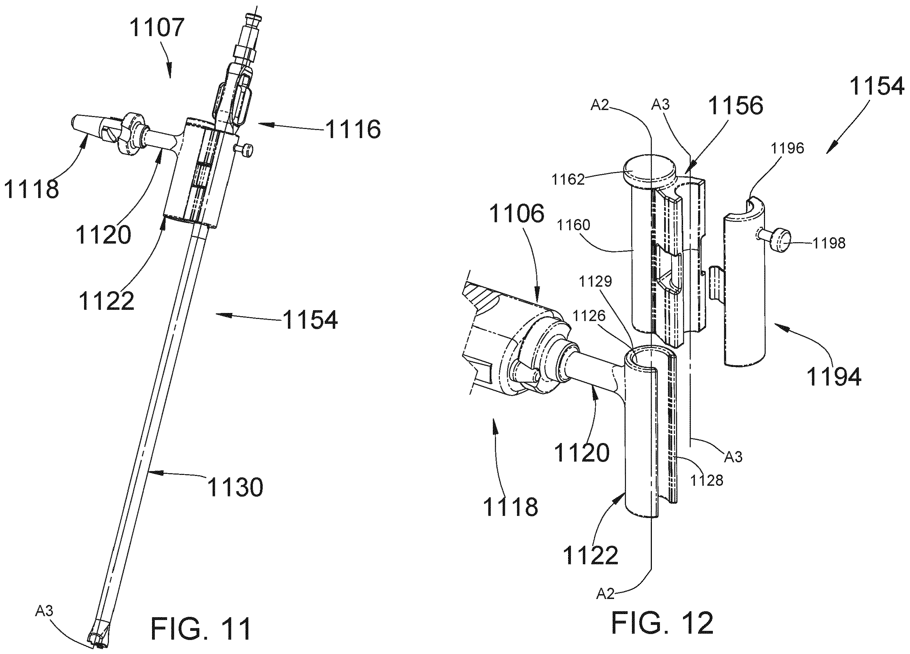

FIG. 11 illustrates an isometric view of an instrument coupler 1107, an instrument adapter 1154, and an implant positioning tool 1116, in accordance with at least one example of this disclosure. FIG. 12 illustrates an isometric view of the instrument coupler 1107 and the instrument adapter 1154, in accordance with at least one example of this disclosure. FIGS. 11 and 12 are discussed below concurrently.

The instrument coupler 1107 can be similar to the coupler 107 discussed above and can include a stem 1118, a neck 1120, and a guide 1122 that includes a bore 1126, a slot 1128, and a proximal portion 1129. The instrument adapter 1154 can include a body 1156, a pin 1160, a stop 1162, and a shell 1194 defining a central bore 1196, together with the body 1156.

The pin 1160 can operate similar to those discussed above to secure the adapter 1154 to the guide 1122. The shell 1194 can be releasably securable to the body 1156 to secure the implant positioning tool 1116 to the instrument adapter 1154. In some examples, the shell 1194 can include a fastener 1198 configured to engage the implant positioning tool 1116 to limit translation of the implant positioning tool 1116 along the axis A3. The axis A3 can be substantially parallel to the axis A2, in some examples.

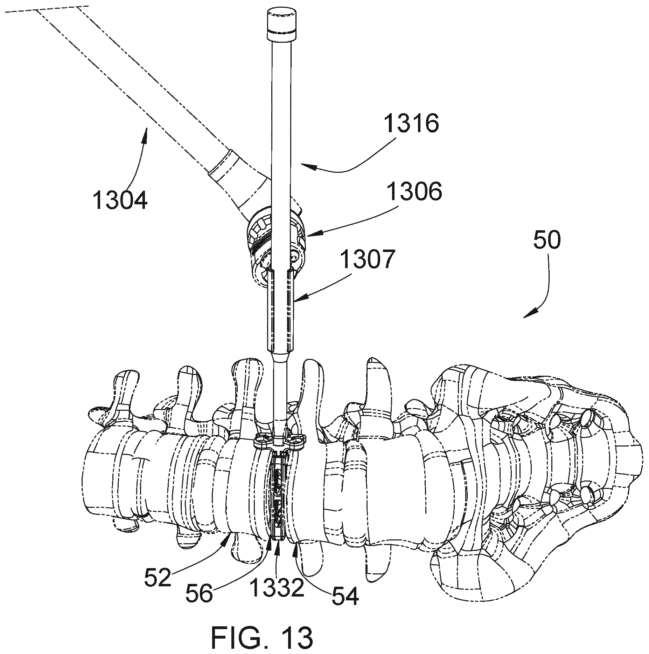

FIG. 13 illustrates an isometric view of a repositionable, lockable surgical arm 1304, in accordance with at least one example of this disclosure. The arm can include an end effector coupler 1306 and a guide 1307 securable to the end effector coupler 1306. The guide 1307 can be configured to receive and retain an implant positioning tool 1316, which can be used for a lower lumbar interbody fusion (LLIF) procedure, as shown in FIG. 13, where the implant positioning tool 1316 is configured to place a cage 1332 in a gap 56 between vertebrae 52 and 54 of a spine 50.

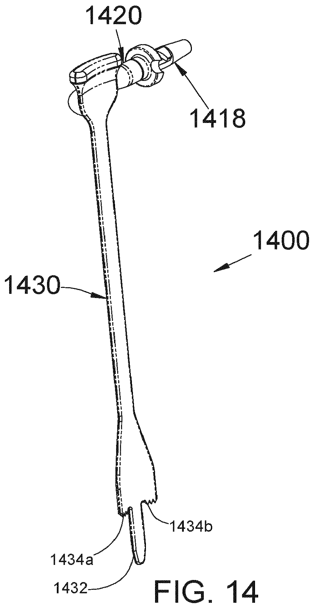

FIG. 14 illustrates an isometric view of an instrument 1400, in accordance with at least one example of this disclosure. The instrument 1400 can be a guide for securing LLIF implants (such as cages) to vertebrae. The instrument 1400 can include a stem 1418 securable to an end effector coupler and a neck 1420 connecting the stem 1418 to a shaft 1430. The instrument 1400 can further include a blade 1432 connected to the shaft and insertable between vertebrae to help limit anterior slipping during insertion and to help achieve proper placement of an implant. Serrations 1334a and 1334b can be connected to the shaft 1430 on either side of the blade 1432 for engaging vertebrae to help maintain a position of the blade 1432 and the instrument 1400 with respect to the vertebrae during insertion of the LLIF cage.

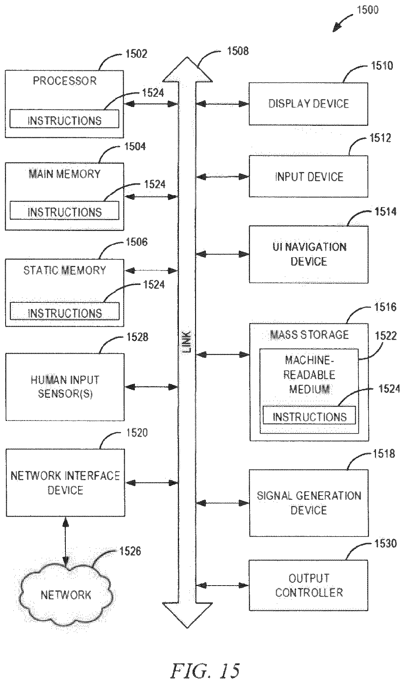

FIG. 15 illustrates a block diagram of an example system or machine 1500 upon which any one or more of the techniques (e.g., methodologies) discussed herein may perform. The machine 1500 can be one or more of the controllers discussed above (120, 110, 210, etc.) Examples, as described herein, may include, or may operate by, logic or a number of components, or mechanisms in the machine 1500. Circuitry (e.g., processing circuitry) is a collection of circuits implemented in tangible entities of the machine 1500 that include hardware (e.g., simple circuits, gates, logic, etc.). Circuitry membership may be flexible over time. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a machine readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine-readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 1500 follow.

In alternative embodiments, the machine 1500 may operate as a standalone device or may be connected (e.g., networked) to other machines. In a networked deployment, the machine 1500 may operate in the capacity of a server machine, a client machine, or both in server-client network environments. In an example, the machine 1500 may act as a peer machine in peer-to-peer (P2P) (or other distributed) network environment. The machine 1500 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine (e.g., computer system) 1500 may include a hardware processor 1502 (e.g., a central processing unit (CPU), a graphics processing unit (GPU), a hardware processor core, or any combination thereof), a main memory 804, a static memory (e.g., memory or storage for firmware, microcode, a basic-input-output (BIOS), unified extensible firmware interface (UEFI), etc.) 1506, and mass storage 1508 (e.g., hard drive, tape drive, flash storage, or other block devices) some or all of which may communicate with each other via an interlink (e.g., bus) 1530. The machine 1500 may further include a display unit 1510 (which can be incorporated into the user interface 110), an alphanumeric input device 1512 (e.g., a keyboard and/or the buttons of the user interface 110), and a user interface (UI) navigation device 1514 (e.g., a mouse). In an example, the display unit 1510, input device 1512 and UI navigation device 1514 may be a touch screen display. The machine 1500 may additionally include a storage device (e.g., drive unit) 1508, a signal generation device 1518 (e.g., a speaker on the user interface 110), a network interface device 1520, and one or more sensors 1516. The machine 1500 may include an output controller 1528, such as a serial (e.g., universal serial bus (USB), parallel, or other wired or wireless (e.g., infrared (IR), near field communication (NFC), etc.) connection to communicate or control one or more peripheral devices (e.g., joint locks and actuators of the arm 104).

Registers of the processor 1502, the main memory 1504, the static memory 1506, or the mass storage 1508 may be, or include, a machine readable medium 1522 on which is stored one or more sets of data structures or instructions 1524 (e.g., software) embodying or utilized by any one or more of the techniques or functions described herein. The instructions 1524 may also reside, completely or at least partially, within any of registers of the processor 1502, the main memory 1504, the static memory 1506, or the mass storage 1508 during execution thereof by the machine 1500. In an example, one or any combination of the hardware processor 1502, the main memory 1504, the static memory 1506, or the mass storage 1508 may constitute the machine readable media 1522. While the machine readable medium 1522 is illustrated as a single medium, the term "machine readable medium" may include a single medium or multiple media (e.g., a centralized or distributed database, and/or associated caches and servers) configured to store the one or more instructions 1524.

The term "machine readable medium" may include any medium that is capable of storing, encoding, or carrying instructions for execution by the machine 1500 and that cause the machine 1500 to perform any one or more of the techniques of the present disclosure, or that is capable of storing, encoding or carrying data structures used by or associated with such instructions. Non-limiting machine readable medium examples may include solid-state memories, optical media, magnetic media, and signals (e.g., radio frequency signals, other photon based signals, sound signals, etc.). In an example, a non-transitory machine readable medium comprises a machine readable medium with a plurality of particles having invariant (e.g., rest) mass, and thus are compositions of matter. Accordingly, non-transitory machine-readable media are machine readable media that do not include transitory propagating signals. Specific examples of non-transitory machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 1524 may be further transmitted or received over a communications network 1526 using a transmission medium via the network interface device 1520 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 1520 may include one or more physical jacks (e.g., Ethernet, coaxial, or phone jacks) or one or more antennas to connect to the communications network 1526. In an example, the network interface device 1520 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1500, and includes digital or analog communications signals or other intangible medium to facilitate communication of such software. A transmission medium is a machine readable medium.

### NOTES AND EXAMPLES

The following, non-limiting examples, detail certain aspects of the present subject matter to solve the challenges and provide the benefits discussed herein, among others.

Example 1 is an instrument coupler receivable in an end effector coupler of a surgical arm, the instrument coupler comprising: a stem including a proximal portion couplable to the end effector coupler; a neck secured to the stem and extending distally therefrom; a guide secured to the neck and configured to receive an implant positioning tool, the guide comprising an elongate body defining a central axis, and defining a bore extending along the central axis, the guide defining a slot extending through the guide and intersecting the bore.

In Example 2, the subject matter of Example 1 includes, wherein edges of the slot receive a cooperative retention structure of the implant positioning tool insertable into the guide, the cooperative retention structure disposed along an elongated portion of the implant positioning tool.

In Example 3, the subject matter of Examples 1-2 includes, wherein the guide includes a proximal end configured to impact a collar of the implant positioning tool to limit translation of the implant positioning tool with respect to the guide.

In Example 4, the subject matter of Examples 1-3 includes, wherein the slot extends along an entirety of a length of the guide.

In Example 5, the subject matter of Examples 1-4 includes, wherein the neck and the stem are substantially coaxial and wherein the central axis is substantially perpendicular to the neck and the stem.

In Example 6, the subject matter of Examples 1-5 includes, wherein the bore of the guide is substantially cylindrical.

In Example 7, the subject matter of Examples 1-6 includes, wherein the bore of the guide includes a flat configured to engage a tool flat of the instrument to orient the instrument with respect to the guide, the stem, and the end effector coupler.

Example 8 is a method for positioning a prosthetic implant, the method comprising: securing an instrument coupler to an end effector coupler of a surgical arm; securing a trial positioning tool to the instrument coupler; moving the trial positioning tool, the instrument coupler, the end effector coupler, and portions of the surgical arm together to position a trial implant of the trial positioning tool in a desired location; locking a position of the instrument coupler, the end effector coupler, and the surgical arm when the trial positioning tool is in the desired location; and securing a prosthetic implant to a vertebra using the instrument coupler in the locked position.

In Example 9, the subject matter of Example 8 includes, inserting an implant positioning tool into a central bore of a guide of the instrument coupler.

In Example 10, the subject matter of Example 9 includes, wherein securing the prosthetic implant includes translating the implant positioning within the guide and with respect to the guide, the end effector coupler, and the surgical arm.

In Example 11, the subject matter of Example 10 includes, wherein translating the implant positioning tool engages the guide to limit non-translational movement of the implant positioning tool with respect to the guide when the implant positioning tool is inserted into the guide and when the instrument coupler is in the locked position.

In Example 12, the subject matter of Example 11 includes, engaging the guide with a collar of the implant positioning tool to limit translation of the implant positioning tool to position the implant in the desired location.

In Example 13, the subject matter of Example 12 includes, releasing the implant from the implant positioning tool.

In Example 14, the subject matter of Example 13 includes, removing the implant positioning tool from the guide.

In Example 15, the subject matter of Examples 8-14 includes, wherein the desired location of the trial implant is between vertebrae of a patient.

In Example 16, the subject matter of Examples 8-15 includes, wherein securing the trial positioning tool to the instrument coupler includes inserting the trial positioning tool into a central bore of a guide of the instrument coupler.

In Example 17, the subject matter of Example 16 includes, wherein securing the trial positioning tool to the central bore of a guide of the instrument coupler includes inserting the trial positioning tool through a slot of the guide that intersects the central bore.

Example 18 is an instrument adapter securable to an instrument and an end effector coupler of a surgical arm, the instrument adapter comprising: a pin insertable into a coupler securable to the end effector coupler; a body secured to the pin and configured to receive an implant positioning tool, the body defining a bore extending along a central axis of the body and defining a slot extending through the body to intersect the bore, the slot configured to receive the implant positioning tool through the slot and into the bore; and a lock ring supported by the body and rotatable between a locked and an unlocked position such that the implant positioning tool is receivable through the slot when the lock ring is in the unlocked positioned and such that the implant positioning tool is prevented from moving through the slot when the lock ring is in the locked position.

In Example 19, the subject matter of Example 18 includes, wherein the slot extends along an entirety of a length of the guide.

In Example 20, the subject matter of Examples 18-19 includes, wherein the central axis is substantially perpendicular to a stem of the coupler.

In Example 21, the subject matter of Examples 18-20 includes, wherein the bore of the guide is cylindrical.

In Example 22, the subject matter of Examples 18-21 includes, wherein the lock ring is rotatable about the central axis within the bore.

In Example 22, the subject matter of Example 22 includes, wherein the body comprises a notch adjacent to the slot and configured to support at least a portion of the lock ring therein.

In Example 23, the subject matter of Example 23 includes, wherein the lock ring comprises a lock ring gap alignable with the slot when the lock ring is in the unlocked position.

In Example 24, the subject matter of Example 24 includes, wherein the lock ring comprises a tab securable within the notch when the lock ring is in the locked position to prevent the lock ring from rotating to a position where the lock ring gap aligns with the slot to prevent the positioning tool from moving through the lock ring gap and the slot.

In Example 26, the subject matter of Examples 18-25 includes, wherein the lock ring includes a channel engageable with a portion of the implant positioning tool to limit rotation of the lock ring to between the locked position and the unlocked position.

Example 27 is an assembly for coupling a surgical instrument to an end effector coupler of a surgical arm, the assembly comprising: an instrument coupler comprising: a stem including a proximal portion couplable to an end effector coupler; a guide secured to the instrument coupler, the guide comprising an elongate body defining a first central axis, and defining a bore extending along the first central axis.

In Example 27, the subject matter of Example 27 includes, a shell releasably securable to the body to secure the implant positioning tool to the instrument adapter.

In Example 29, the subject matter of Examples 27-27 includes, the body defining a slot extending through the body, the slot configured to receive the implant positioning tool into the bore.

In Example 29, the subject matter of Example 29 includes, a lock ring supported by the body and rotatable between a locked and an unlocked position, such that the implant positioning tool is receivable through the slot when the lock ring is in the unlocked positioned and is prevented from moving through the slot when the lock ring is in the locked position.

In Example 31, the subject matter of Examples 27-30 includes, a neck connecting the stem to the guide.

In Example 32, the subject matter of Examples 27-31 includes, wherein the guide defines a slot extending through the guide and intersecting the bore.

In Example 32, the subject matter of Example 32 includes, wherein edges of the slot receive a cooperative retention structure of the instrument adapter insertable into the guide the cooperative retention structure disposed along an elongated portion of the implant positioning tool.

In Example 34, the subject matter of Examples 27-33 includes, wherein a proximal end of the guide is configured to impact a collar of the instrument adapter to limit translation of the instrument adapter with respect to the guide.

In Example 35, the subject matter of Examples 27-34 includes, wherein the body defines a slot extending through the body to intersect the bore, the slot configured to receive the implant positioning tool through the slot and into the bore, and wherein the instrument adapter includes a lock ring supported by the body and rotatable between a locked and an unlocked position such that the implant positioning tool is receivable through the slot when the lock ring is in the unlocked positioned and such that the implant positioning tool is prevented from moving through the slot when the lock ring is in the locked position.

In Example 36, the subject matter of Examples 34-34 includes, wherein the lock ring is rotatable about the central axis within the bore.

In Example 36, the subject matter of Example 36 includes, wherein the body comprises a notch adjacent to the slot and configured to support at least a portion of the lock ring therein.

In Example 37, the subject matter of Example 37 includes, wherein the lock ring comprises a lock ring gap alignable with the slot when the lock ring is in the unlocked position.

In Example 38, the subject matter of Example 38 includes, wherein the lock ring comprises a tab securable within the notch when the lock ring is in the locked position to prevent the lock ring from rotating to a position where the lock ring gap aligns with the slot to prevent the positioning tool from moving through the lock gap slot and the slot.

In Example 40, the subject matter of Examples 35-39 includes, wherein the lock ring includes a channel engageable with a portion of the implant positioning tool to limit rotation of the lock ring to between the locked position and the unlocked position.

Example 41 is at least one machine-readable medium including instructions that, when executed by processing circuitry, cause the processing circuitry to perform operations to implement of any of Examples 1-40.

Example 42 is an apparatus comprising means to implement of any of Examples 1-40.

Example 43 is a system to implement of any of Examples 1-40.

Example 44 is a method to implement of any of Examples 1-40.

In Example 45, the apparatuses or method of any one or any combination of Examples 1 - 44 can optionally be configured such that all elements or options recited are available to use or select from.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. An instrument adapter securable to an instrument and an end effector coupler of a surgical arm, the instrument adapter comprising:
a pin insertable into a coupler securable to the end effector coupler;
a body secured to the pin and configured to receive an implant positioning tool, the body defining a bore extending along a central axis of the body and defining a slot extending through the body to intersect the bore, the slot configured to receive the implant positioning tool through the slot and into the bore; and
a lock ring supported by the body and rotatable between a locked and an unlocked position such that the implant positioning tool is receivable through the slot when the lock ring is in the unlocked positioned and such that the implant positioning tool is prevented from moving through the slot when the lock ring is in the locked position.

2. The instrument adapter of claim 1, wherein the slot extends along an entirety of a length of a guide of the coupler.

3. The instrument adapter of any of claims 1-2, wherein the central axis is substantially perpendicular to a stem of the coupler.

4. The instrument adapter of any of claims 1-3, wherein the bore of the body is cylindrical.

5. The instrument adapter of any of claims 1-4, wherein the lock ring is rotatable about the central axis within the bore.

6. The instrument adapter of claim 5, wherein the body comprises a notch adjacent to the slot and configured to support at least a portion of the lock ring therein.

7. The instrument adapter of claim 6, wherein the lock ring comprises a lock ring gap alignable with the slot when the lock ring is in the unlocked position.

8. The instrument adapter of claim 7, wherein the lock ring comprises a tab securable within the notch when the lock ring is in the locked position to prevent the lock ring from rotating to a position where the lock ring gap aligns with the slot to prevent the positioning tool from moving through the lock ring gap and the slot.

9. The instrument adapter of claim 1, wherein the lock ring includes a channel engageable with a portion of the implant positioning tool to limit rotation of the lock ring to between the locked position and the unlocked position.

10. The instrument adapter of claim 9, wherein the portion of the implant positioning tool engageable with the lock ring channel is a rotation pin.

11. The instrument adapter of claim 10, wherein the rotation pin is secured to an outer surface of a shaft of the implant positioning tool.

12. An assembly for coupling a surgical instrument to an end effector coupler of a surgical arm, the assembly comprising the instrument adapter of claim 1.

13. The assembly of claim 12, wherein the assembly incudes the coupler, the coupler comprising:
a stem including a proximal portion couplable to an end effector coupler; and
a guide secured to the instrument coupler, the guide comprising an elongate body defining a guide central axis, and the guide defining a guide bore extending along the guide central axis.

14. The assembly of claim 13, wherein guide bore is configured to receive the pin of the instrument adapter therein.

15. An instrument coupler receivable in an end effector coupler of a surgical arm, the instrument coupler comprising:
a stem including a proximal portion couplable to the end effector coupler;
a neck secured to the stem and extending distally therefrom; and
a guide secured to the neck and configured to receive an implant positioning tool, the guide comprising an elongate body defining a central axis, and defining a bore extending along the central axis, the guide defining a slot extending through the guide and intersecting the bore.

16. The instrument of claim 15, wherein edges of the slot receive a cooperative retention structure of the implant positioning tool insertable into the guide, the cooperative retention structure disposed along an elongated portion of the implant positioning tool.

17. The instrument of any of claims 15 and 16, wherein the guide includes a proximal end configured to impact a collar of the implant positioning tool to limit translation of the implant positioning tool with respect to the guide.

18. The instrument of any of claims 15 through 17, wherein the slot extends along an entirety of a length of the guide.

19. The instrument of any of claims 15 through 18, wherein the neck and the stem are substantially coaxial and wherein the central axis is substantially perpendicular to the neck and the stem.

20. The instrument of any of claims 15 through 19, wherein the bore of the guide is substantially cylindrical.

21. The instrument of any of claims 15 through 20, wherein the bore of the guide includes a flat configured to engage a tool flat of the instrument to orient the instrument with respect to the guide, the stem, and the end effector coupler.

22. A method for positioning a prosthetic implant, the method comprising:
securing an instrument coupler to an end effector coupler of a surgical arm;
securing a trial positioning tool to the instrument coupler;
moving the trial positioning tool, the instrument coupler, the end effector coupler, and portions of the surgical arm together to position a trial implant of the trial positioning tool in a desired location;
locking a position of the instrument coupler, the end effector coupler, and the surgical arm when the trial positioning tool is in the desired location; and
securing a prosthetic implant to a vertebra using the instrument coupler in the locked position.

23. The method of claim 22, further comprising:
inserting an implant positioning tool into a central bore of a guide of the instrument coupler.

24. The method of claim 23, wherein securing the prosthetic implant includes translating the implant positioning within the guide and with respect to the guide, the end effector coupler, and the surgical arm.

25. The method of claim 24, wherein translating the implant positioning tool engages the guide to limit non-translational movement of the implant positioning tool with respect to the guide when the implant positioning tool is inserted into the guide and when the instrument coupler is in the locked position.

26. The method of claim 25, further comprising:
engaging the guide with a collar of the implant positioning tool to limit translation of the implant positioning tool to position the implant in the desired location.

27. The method of claim 26, further comprising:
releasing the implant from the implant positioning tool.

28. The method of claim 27, further comprising:
removing the implant positioning tool from the guide.

29. The method of any of claims 22-28, wherein the desired location of the trial implant is between vertebrae of a patient.
